# EUROPEAN PATENT SPECIFICATION

(11) **EP 3 996 759 B1**
(45) Date of publication and mention of the grant of the patent: **09.08.2023**
(21) Application number: 20736384.7
(22) Date of filing: 09.07.2020
(51) Int. Cl.: A61L 15/42, A61L 15/64, A61L 15/22

(54) **BIOCOMPATIBLE, FLEXIBLE, HAEMOSTATIC SHEET**
BIOKOMPATIBLE FLEXIBLE HÄMOSTATISCHE FOLIE
FEUILLE HÉMOSTATIQUE BIOCOMPATIBLE ET FLEXIBLE

(30) Priority: 12.07.2019 EP 19186026
(43) Date of publication of application: 18.05.2022
(60) Divisional application: 23179003.1
(73) Proprietor: Cilag GmbH International, 6300 Zug (CH); Ethicon, Inc., Somerville, New Jersey 08876 (US)
(72) Inventor: KEEREWEER, Abraham Reinier, 6534 AT Nijmegen (NL); FÉLIX LANAO, Rosa Pilar, 6534 AT Nijmegen (NL); OPSTEEN, Joost, 6534 AT Nijmegen (NL); BENDER, Johannes Caspar Mathias Elizabeth, 6534 AT Nijmegen (NL); LLANOS, Gerard, Somerville, NJ 08876 (US)
(74) Representative: Nederlandsch Octrooibureau
(86) International application number: PCT/EP2020/069443
(87) International publication number: WO 2021/009015

(56) References cited:
- WO-A1-2016/056901
- WO-A2-2010/059280
- WO-A2-2013/053759
- MARCEL A. BOERMAN ET AL: "Next Generation Hemostatic Materials Based on NHS-Ester Functionalized Poly(2-oxazoline)s", BIOMACROMOLECULES, vol. 18, no. 8, 25 July 2017 (2017-07-25), pages 2529-2538, XP055655362, US ISSN: 1525-7797, DOI: 10.1021/acs.biomac.7b00683

## Description

### TECHNICAL FIELD OF THE INVENTION

The present invention relates to a biocompatible, flexible, haemostatic sheet that can suitably be used to minimise haemorrhage during surgical procedures. This haemostatic sheet comprises:
- a cohesive fibrous carrier structure comprising a three-dimensional interconnected interstitial space and,
- distributed within the interstitial space, reactive polymer particles comprising (i) a water-soluble electrophilic polymer carrying at least three reactive electrophilic groups, said reactive electrophilic groups being capable of reacting with amine groups in tissue and blood under the formation of a covalent bond and (ii) a nucleophilic cross-linking agent that contains at least two reactive nucleophilic groups that are capable of reacting with the reactive electrophilic groups of the electrophilic polymer under the formation of a covalent bond.

### BACKGROUND OF THE INVENTION

One of the main challenges during surgical procedures on parenchymatous tissue is to attain control over bleeding. Suture control, electrocautery, and ultrasonic sealing often do not suffice during operations on, for example, liver or kidneys. As a result, procedures like hepatic resections or partial nephrectomy require an alternative approach to control bleeding. For this purpose, a wide range of topical haemostatic products has been developed and are clinically available.

Lewis et al. (Control of bleeding in surgical procedures: critical appraisal of HEMOPATCH (Sealing Hemostat), Dove Press Journal: Medical Devices Evidence and Research, 22 Dec. 2015, 1-9) describe a haemostatic pad (HEMOPATCH) that is composed of a synthetic, protein-reactive monomer and a collagen backing. The active side is covered with the protein-reactive monomer: N-hydroxysuccinimide functionalized polyethylene glycol (NHS-PEG). NHS-PEG rapidly affixes the collagen pad to tissue to promote and maintain hemostasis.

Schuhmacher et al. (Safety and effectiveness of a synthetic hemostatic patch for intraoperative soft tissue bleeding, Med Devices (Auckl). 2015; 8: 167-174) describe a hemostatic patch (Veriset^{™}) that is composed of an absorbable backing material, oxidized cellulose and polyethylene glycol hydrogel. Veriset^{™} hemostatic patch is provided as a ready-to-use patch that is applied polyethylene glycol-side down to the bleeding site.

Boerman et al. (Next Generation Hemostatic Materials Based on NHS-Ester Functionalized Poly(2-oxazoline)s, Biomacromolecules (2017), 18, 2529-2538) describe a synthetic, nonbioactive hemostatic product that is obtained by coating N-hydroxysuccinimide ester (NHS)-functional poly(2-oxazoline)s (NHS-POx) onto gelatin patches, which acts by formation of covalent cross-links between polymer, host blood proteins, gelatin and tissue to seal the wound site and prevent haemorrhage during surgery.

US 2010/0233246 describes a biocompatible polymer device comprising a collagen sponge or sheet impregnated with a two-part reactive polyethylene glycol powder, wherein said reactive powder comprises a first polyethylene glycol having nucleophilic groups and a second polyethylene glycol having electrophilic groups, wherein the polyethylene glycol powder remains unreactive in the dry state.

WO 2010/059280 describes an anhydrous fibrous sheet comprising a first component of fibrous polymer, said polymer containing electrophilic groups or nucleophilic groups, and a second component capable of crosslinking the first component when said sheet is exposed to an aqueous medium in contact with biological tissue to form a crosslinked hydrogel that is adhesive to the biological tissue; wherein:
a) wherein the second component is a fibrous polymer having a backbone structure the same as or different from the fibrous polymer of the first component and containing electrophilic groups if the first component contains nucleophilic groups or containing nucleophilic groups if the first component contains electrophilic groups;
b) the second component is a coating on the fibrous polymer of the first component and wherein said coating contains electrophilic groups if the first component contains nucleophilic groups or nucleophilic groups if the first component contains electrophilic groups; or
c) the second component is a dry powder dispersed and entrapped within interstices of the fibrous polymer of the first component, wherein said powder contains electrophilic groups if the first component contains nucleophilic groups or nucleophilic groups if the first component contains electrophilic groups.

US 2011/0250257 describes an anhydrous fibrous sheet comprising a first component of fibrous polymer, said polymer containing electrophilic groups or nucleophilic groups, and a second component capable of crosslinking the first component when said sheet is exposed to an aqueous medium in contact with biological tissue to form a crosslinked hydrogel that is adhesive to the biological tissue; wherein the second component is a fibrous polymer and containing electrophilic groups if the first component contains nucleophilic groups or containing nucleophilic groups if the first component contains electrophilic groups; or the second component is a coating on the fibrous polymer of the first component, wherein said coating contains electrophilic groups if the first component contains nucleophilic groups or nucleophilic groups if the first component contains electrophilic groups; or the second component is a dry powder dispersed and entrapped within interstices of the fibrous polymer of the first component, wherein said powder contains electrophilic groups if the first component contains nucleophilic groups or nucleophilic groups if the first component contains electrophilic groups.

WO 2011/124640 describes a method of manufacturing a hemostatic sponge comprising:
a) providing a sponge comprising a matrix of a biomaterial in dried form,
b) providing one reactive polymeric material in the form of dry powder,
c) contacting a) and b) so that the material of b) is present on at least one surface of said sponge, and
d) fixing the material of b) on the sponge of a).

Fixation can be achieved by melting for a sufficiently long time period.

WO 2012/057628 describes a tissue-adhesive medical product comprising at least 1% by weight of dry matter of an electrophilically activated polyoxazoline (EL-POx), said EL-POx comprising at least 2 reactive electrophilic groups, including at least one pendant electrophilic group. Besides EL-POx, the medical product may contain a nucleophilically activated polyoxazoline (NU-POx). Examples of tissue-adhesive products include adhesive tissue tape, tissue sealant, haemostatic porous material and implants.

WO 2016/056901 describes an adhesive haemostatic product selected from a coated mesh, a coated foam or a coated powder, said haemostatic product comprising:
- a porous solid substrate having a porosity of at least 5 vol.% and comprising an outer surface that comprises a nucleophilic polymer containing reactive nucleophilic groups;
- an adhesive coating that covers at least a part of the solid substrate, said coating comprising an electrophilically activated polyoxazoline (EL-POx), said EL-POx containing on average at least 1 reactive electrophilic group.

The adhesive haemostatic product is produced by a process comprising the steps of providing a porous solid substrate; coating the substrate with a coating liquid that comprises EL-POx and a solvent; and removing the solvent.

### SUMMARY OF THE INVENTION

The inventors have developed a biocompatible, flexible, haemostatic sheet that is particularly suited for preventing haemorrhage during laparoscopic surgical procedures.

The haemostatic sheet according to the present invention comprises a cohesive fibrous carrier structure that holds small particles comprising a polymer that is capable of covalently binding with host blood proteins and tissue, and which thereby induces haemostatis and/or tissue-adhesion. Thus, one aspect of the invention relates to a biocompatible, flexible, haemostatic sheet comprising:
- a cohesive fibrous carrier structure comprising a three-dimensional interconnected interstitial space; and
- distributed within the interstitial space, a plurality of reactive polymer particles comprising (i) a water-soluble electrophilic polymer carrying at least 3 reactive electrophilic groups that are capable of reacting with amine groups in tissue and blood under the formation of a covalent bond and (ii) a nucleophilic cross-linking agent that contains at least two reactive nucleophilic groups that are capable of reacting with the reactive electrophilic groups of the electrophilic polymer under the formation of a covalent bond, said reactive polymer particles having a diameter in the range of 0.5-100 µm and being present in an amount of at least 3% by weight of the fibrous carrier structure.

The haemostatic sheet of the present invention comprises a cohesive fibrous carrier structure that readily absorbs blood as blood can penetrate the interstitial space. This fibrous carrier structure can easily be impregnated with reactive polymer particles. Unlike impregnation with liquids, such dry impregnation does not affect the structural integrity or mechanical properties of the carrier structure. When blood is absorbed by the haemostatic sheet of the present invention, the water-soluble electrophilic polymer in the reactive polymer particles starts dissolving as soon as these particles are 'wetted' by the blood, thereby allowing the electrophilic polymer to react with reactive nucleophilic groups in the blood and tissue, as well as with reactive nucleophilic groups of the nucleophilic cross-linking agent, thereby inducing blood coagulation and tissue sealing, both of which contribute to haemostasis.

Although the inventors do not wish to be bound by theory, it is believed that the advantageous properties of the haemostatic sheet of the present invention can be attributed to the fact that the reactive polymer particles are distributed throughout the fibrous carrier structure creating a minimum of bending friction and also to the fact that, due to the small particles size, these reactive polymer particles dissolve rapidly when they come into contact with blood or other aqueous bodily fluids. Thus, upon application of the sheet onto a wound site, rapid covalent cross-linking occurs between on the one hand the reactive electrophilic polymer, and on the other hand blood proteins, tissue and the nucleophilic cross-linking agent, leading to the formation of a hydrogel which seals off the wound surface and stops the bleeding and which can provide strong adhesion of the fibrous sheet to the tissue, due to the formation of covalent bonds between reactive electrophilic groups in the hydrogel and amine/thiol groups in the tissue. The fibrous carrier structure provides mechanical strength during and after application, and prevents excessive swelling.

By combining the polymer carrying reactive electrophilic groups and the nucleophilic crosslinking agent in a single particle, it is ensured that these two reactive components can be homogeneously distributed throughout the haemostatic sheet, that no segregation occurs during transport and handling, and that these components can react immediately with each other when the particles come into contact with blood.

Due to its flexibility, the haemostatic sheet of the present invention can suitably be applied to irregularly shaped bleeding sites. The haemostatic sheet may be applied layer on layer if an already applied sheet does not fully stop the bleeding.

Another aspect of the present invention relates to a method of preparing a haemostatic sheet, said method comprising:
- providing a sheet of cohesive fibrous carrier structure as defined above;
- providing reactive polymer particles as defined above; and
- distributing the reactive polymer particles within the interstitial space of the fibrous carrier structure.

### DETAILED DESCRIPTION OF THE INVENTION

Accordingly, one aspect of the invention relates to a biocompatible, flexible, haemostatic sheet comprising:
- a cohesive fibrous carrier structure comprising a three-dimensional interconnected interstitial space; and
- distributed within the interstitial space, a plurality of reactive polymer particles comprising (i) a water-soluble electrophilic polymer carrying at least three reactive electrophilic groups that are capable of reacting with amine groups in tissue and blood under the formation of a covalent bond and (ii) a nucleophilic cross-linking agent that contains at least two reactive nucleophilic groups that are capable of reacting with the reactive electrophilic groups of the electrophilic polymer under the formation of a covalent bond, said reactive polymer particles having a diameter in the range of 0.5-100 µm and being present in an amount of at least 3% by weight of the fibrous carrier structure.

The term "haemostatic sheet" as used herein, unless indicated otherwise, refers to a sheet having the ability to stop bleeding from damaged tissue. The haemostatic sheet of the present invention may achieve haemostasis by turning blood into a gel and/or by forming a seal that closes off the wound site.

The term "tissue-adhesive" as used herein refers to the ability of the haemostatic sheet to cling to tissue due to the formation of covalent bonds between the sheet and the tissue. Formation of these covalent bonds typically requires the presence of water.

The term "interstitial space" as used herein refers to the void ("empty") space within the fibrous carrier structure. The interstitial space within the fibrous carrier structure allows the introduction of reactive polymer particles into the structure. Also blood and other bodily fluids can enter the interstitial space, allowing the water-soluble electrophilic polymer and the nucleophilic cross-linking agent within the reactive polymer particles to dissolve.

The concentration of reactive polymer particles having a diameter in the range of 0.5-100 µm is expressed in % by weight of the fibrous carrier structure *per se,* i.e. without the reactive polymer particles.

The "water-soluble electrophilic polymer carrying reactive electrophilic groups" that is employed in accordance with the present invention carries at least three reactive groups that are capable of reacting with amine groups in tissue and blood under the formation of a covalent bond. This water-soluble electrophilic polymer has a molecular weight of at least 1 kDa and a solubility in distilled water of 20°C of at least 50 g/L.

The term "water absorption capacity" as used herein is a measure of the capability of the haemostatic sheet to absorb water. The water absorption capacity is determined by weighing a sample of the dry sheet (weight = W_{d}) followed by immersion of the sample into distilled water (37°C) for 45 minutes. Next, the sample is removed from the water and water clinging to the outside of the substrate is removed, following which the sample is weighed again (weight = W_{w}). The water absorption capacity = 100% × (W_{w}-W_{d})/W_{d}. The water adsorption capacity is indicative of the porosity of the substrate as well as of its ability to swell in the presence of water.

The term "collagen" as used herein refers the main structural protein in the extracellular space of various connective tissues in animal bodies. Collagen forms a characteristic triple helix of three polypeptide chains. Depending upon the degree of mineralization, collagen tissues may be either rigid (bone) or compliant (tendon) or have a gradient from rigid to compliant (cartilage). Unless indicated otherwise, the term "collagen" also encompasses modified collagens other than gelatin.

The term "gelatin" as used herein refers to a mixture of peptides and proteins produced by partial hydrolysis of collagen extracted from the skin, bones, and connective tissues of animals such as domesticated cattle, chicken, pigs, and fish. During hydrolysis, the natural molecular bonds between individual collagen strands are broken down into a form that rearranges more easily.

The term "polyoxazoline" as used herein refers to a poly(N-acylalkylenimine) or a poly(aroylalkylenimine) and is further referred to as POx. An example of POx is poly(2-ethyl-2-oxazoline). The term "polyoxazoline" also encompasses POx copolymers.

The reactive polymer particles may be homogeneously distributed within the interstitial space of the fibrous carrier structure in the sense that the particle density is essentially the same throughout the carrier structure. The reactive polymer particles may also be unevenly distributed throughout the carrier structure. For instance, if the haemostatic sheet is prepared in the form of a laminate of thin layers of fibrous carrier structure and layers of reactive polymer particles, the reactive polymer particle density within the sheet may fluctuate. For certain applications it may be advantageous if the reactive polymer particle density shows a gradient, e.g. in that the density of reactive particles is lowest near the side of the sheet that is meant to applied onto a bleeding wound and highest near the other side of the sheet.

The diameter distribution of the reactive polymer particles may suitably be determined by means of laser diffraction using a Malvern Mastersizer 2000 in combination with the Stainless Steel Sample Dispersion Unit. The sample dispersion unit is filled with approx. 120 ml of cyclohexane, which is stabilized for 5 to 10 minutes at a stirring speed of 1800 rpm, followed by a background measurement (blanc measurement). The sample tube is shaken and turned horizontally for 20 times. Next, about 50 mg is dispersed in the sample dispersion unit containing the cyclohexane. After the sample is introduced in the dispersion unit, the sample is stirred for one and a half minute at 1800 rpm to ensure that all particles are properly dispersed, before carrying out the measurement. No ultrasonic treatment is performed on the dispersed particles. Mean particle size is expressed as D [4,3], the volume weighted mean diameter (ΣniDi⁴)/(ΣniDi³).

In a particularly preferred embodiment, unlike the fibrous tissue sealant described in US 2011/0250257, the haemostatic sheet of the present invention does not form a hydrogel, i.e. a water-swellable polymeric matrix that can absorb a substantial amount of water to form an elastic gel.

According to a particularly preferred embodiment, the haemostatic sheet of the present invention is bioabsorbable, meaning that the carrier structure, the reactive polymer particles and any other components of the haemostatic sheet are eventually absorbed in the body. Absorption of the carrier structure and reactive polymer particles typically requires chemical decomposition (e.g. hydrolysis) of polymers contained therein. Complete bioabsorption of the haemostatic sheet by the human body is typically achieved in 1 to 10 weeks, preferably in 2 to 8 weeks.

In accordance with another preferred embodiment, the cohesive fibrous carrier structure is water-resistant. Here, the term "water-resistant" means that the cohesive fibrous carrier structure is not water soluble and does not disintegrate in water to form a colloidal dispersion, at neutral pH conditions (pH 7) and a temperature of 37°C.

The haemostatic sheet of the present invention typically has a non-compressed mean thickness of 0.5-25 mm. More preferably, the non-compressed mean thickness is in the range of 1-10 mm, most preferably in the range of 1.5-5 mm.

The dimensions of the haemostatic sheet preferably are such that the top and bottom of the sheet each have a surface area of at least 2 cm², more preferably of at least 10 cm² and most preferably of 25-50 cm². Typically, the sheet is rectangular in shape and has a length of 25-200 mm, an a width of 25-200 mm.

The haemostatic sheet preferably has a non-compressed density of less than 200 mg/cm³, more preferably of less than 150 mg/cm³ and most preferably of 10-100 mg/cm³.

In one embodiment of the invention the reactive polymer particles are homogeneously distributed within the interstitial space of the fibrous carrier structure. In another embodiment of the invention the haemostatic sheet is a laminate comprising alternating layers of fibrous carrier structure and layers of the reactive polymer particles. In the latter embodiment, reactive polymer particles preferably have entered the layers of fibrous carrier structure that separate the layers of reactive polymer particles.

The haemostatic sheet of the present invention preferably is essentially anhydrous. Typically, the haemostatic sheet has a water content of not more than 5 wt.%, more preferably of not more than 2 wt.% and most preferably of not more than 1 wt.%.

The water absorption capacity of the haemostatic sheet preferably is at least 50%, more preferably lies in the range of 100% to 800%, most preferably in the range of 200% to 500%.

The haemostatic sheet of the present invention is preferably sterile.

The use of a fibrous carrier structure in the haemostatic sheet of the present invention offers the advantage that the reactive polymer particles can be homogeneously distributed throughout this carrier structure without difficulty. Such a homogeneous distribution is much more difficult to achieve in, for instance, foamed carrier structures.

The fibres in the fibrous carrier structure preferably have a mean diameter of 1-500 µm, more preferably of 2-300 µm and most preferably of 5-200 µm. The mean diameter of the fibres can suitably be determined using a microscope.

Typically, at least 50 wt.%, more preferably at least 80 wt.% of the fibres in the fibrous carrier structure have a diameter of 1-300 µm and a length of at least 1 mm.

Preferably, at least 50 wt.%, more preferably at least 80 wt.% of the fibres in the fibrous carrier structure have an aspect ratio (ratio of length to diameter) of at least 1000.

The fibrous carrier structure that is employed in accordance with the present invention preferably is a felt structure, a woven structure or a knitted structure. Most preferably, the fibrous carrier structure is a felt structure. Here the term "felt structure" refers to a structure that is produced by matting and pressing fibres together to form a cohesive material.

According to a particularly preferred embodiment, the fibrous carrier structure is biodegradable.

The fibrous carrier structure preferably comprises fibres containing a fibre polymer selected from cellulose, modified cellulose, carboxymethyldextran, poly(lactic-co-glycolic acid) (PLGA), sodium hyaluronate/carboxy methylcellulose, polyvinyl alcohol and combinations thereof. More preferably, the fibrous carrier structure comprises fibres containing modified cellulose, even more preferably oxidised cellulose and most preferably oxidised regenerated cellulose.

The fibrous carrier structure preferably comprises at least 50 wt.%, more preferably at least 80 wt.% and most preferably at least 90 wt.% of fibres containing the aforementioned fibre polymer.

In a preferred embodiment, the fibrous carrier structure comprises at least 50 wt.%, more preferably at least 80 wt.% and most preferably at least 90 wt.% fibres containing at least 50 wt.% of the fibre polymer.

In an even more preferred embodiment, the fibrous carrier structure comprises at least 50 wt.%, more preferably at least 80 wt.% and most preferably at least 90 wt.% fibres made from modified cellulose, more preferably made from oxidized cellulose, and most preferably made from oxidised regenerated cellulose.

The fibres contained in the fibrous carrier structure preferably do not contain a polymer carrying reactive nucleophilic groups that are capable of reacting with the reactive electrophilic groups of the water-soluble electrophilic polymer under the formation of a covalent bond. Likewise, the fibres contained in the fibrous carrier structure preferably do not contain a nucleophilic cross-linking agent containing two or more reactive nucleophilic groups that are capable of reacting with the reactive electrophilic groups of the water-soluble electrophilic polymer under the formation of a covalent bond.

The invention encompasses the use of both regenerated oxidized cellulose and the use of non-regenerated oxidized cellulose, the former being preferred. Oxidized cellulose can be produced from cellulose by the action of an oxidizing agent. Oxidized cellulose contains carboxylic acids in addition to the original hydroxyl groups of the starting material. Prior to oxidation, the cellulose can remain non-regenerated with unorganized fibres or it can be regenerated to form organized fibres. When cellulose fibres are treated with dinitrogen tetroxide, hydroxyl groups are oxidized into carboxylic acid groups yielding a polyuronic acid.

Preferred fibrous carrier structures have an open pore structure with a permeability to air of at least 0.1 L/min × cm², more preferably of at least 0.5 L/min × cm². The air permeability is determined in accordance with EN ISO 9237:1995 (Textiles - Determination of the permeability of fabrics to air).

The fibres in the fibrous carrier structure can be produced by means of methods known in the art, such as electrospinning, electro-blown spinning and high speed rotary sprayer spinning. Production of fibrous carrier structure by means of high speed rotary sprayer spinning is described in US 2015/0010612. It is also possible to use commercially available haemostatic fibrous sheets as the fibrous carrier structure. Examples of suitable commercial products include SURGICEL SNoW^{™} (ex Johnson & Johnson Medical), NU-KNIT^{™} (ex Johnson & Johnson Medical) and EVARREST^{®} (ex Ethicon).

The reactive polymer particles are preferably present in the haemostatic sheet of the present invention in an amount of 5-90%, more preferably 10-80%, even more preferably 20-75% and most preferably 50-70%, by weight of the fibrous carrier structure.

The reactive polymer particles preferably contain at least 10 wt.% of the water-soluble electrophilic polymer. More preferably, the reactive polymer particles contain at least 50 wt.%, more preferably at least 90 wt.% of the electrophilic polymer.

The reactive polymer particles that are distributed within the interstitial space of the fibrous carrier structure preferably have a volume weighted mean particle size in the range of 2-75 µm, more preferably in the range of 1-50 µm and most preferably in the range of 1-25 µm.

The reactive polymer particles of the present invention may be prepared in various ways, e.g. by milling, by spray drying a polymeric solution, by freeze drying, by spray chilling a polymeric melt, by granulating a powder mixture, or by fluidised bed coating.

The water-soluble electrophilic polymer typically has a molecular weight of at least 2 kDa, more preferably of at least 5 kDa and most preferably of 10-100 kDa.

The water-soluble electrophilic polymer preferably has a solubility in distilled water of 20°C of at least 100 g/L, more preferably of at least 200 g/L.

The water-soluble electrophilic polymer that is employed in accordance with the present invention, preferably contains at least 4 reactive electrophilic groups, more preferably at least 8 reactive electrophilic groups, even more preferably at least 16 reactive electrophilic groups and most preferably at least 32 reactive electrophilic groups.

The reactive polymer particles in the haemostatic sheet preferably comprise a water-soluble electrophilic polymer that carries reactive electrophilic groups selected from carboxylic acid esters, sulfonate esters, phosphonate esters, pentafluorophenyl esters, p-nitrophenyl esters, p-nitrothiophenyl esters, acid halide groups, anhydrides, ketones, aldehydes, isocyanato, thioisocyanato, isocyano, epoxides, activated hydroxyl groups, olefins, glycidyl ethers, carboxyl, succinimidyl esters, sulfo succinimidyl esters, maleimido (maleimidyl), ethenesulfonyl, imido esters, aceto acetate, halo acetal, orthopyridyl disulfide, dihydroxyphenyl derivatives, vinyl, acrylate, acrylamide, iodoacetamide and combinations thereof. More preferably, the reactive electrophilic groups are selected from carboxylic acid esters, sulfonate esters, phosphonate esters, pentafluorophenyl esters, p-nitrophenyl esters, p-nitrothiophenyl esters, acid halide groups, anhyinidrides, ketones, aldehydes, isocyanato, thioisocyanato, isocyano, epoxides, activated hydroxyl groups, glycidyl ethers, carboxyl, succinimidyl esters, sulfo succinimidyl esters, imido esters, dihydroxy-phenyl derivatives, and combinations thereof. Even more preferably, the reactive electrophilic groups are selected from halo acetals, orthopyridyl disulfide, maleimides, vinyl sulfone, dihydroxyphenyl derivatives, vinyl, acrylate, acrylamide, iodoacetamide, succinimidyl esters and combinations thereof. Most preferably, the reactive electrophilic groups are selected from maleimides, vinyl, acrylate, acrylamide, succinimidyl esters, sulfo succinimidyl esters and combinations thereof.

Examples of succinimidyl esters that may be employed include succinimidyl glutarate, succinimidyl propionate, succinimidyl succinamide, succinimidyl carbonate, disuccinimidyl suberate, bis(sulfosuccinimidyl) suberate, dithiobis(succinimidylpropionate), bis(2-succinimidooxycarbonyloxy) ethyl sulfone, 3,3'-dithiobis(sulfosuccinimidyl-propionate), succinimidyl carbamate, sulfosuccinimidyl(4-iodoacetyl)aminobenzoate, bis(sulfosuccinimidyl) suberate, sulfosuccinimidyl-4-(N-maleimidomethyl)-cyclohexane-l-carboxylate, dithiobis-sulfosuccinimidyl propionate, disulfo-succinimidyl tartarate; bis[2-(sulfosuccinimidyloxycarbonyloxyethylsulfone)], ethylene glycol bis(sulfosuccinimiclylsuccinate), dithiobis-(succinimidyl propionate).

Examples of dihydroxyphenyl derivatives that may be employed include dihydroxyphenylalanine, 3,4-dihydroxyphenylalanine (DOPA), dopamine, 3,4-dihydroxyhydroccinamic acid (DOHA) , norepinephrine, epinephrine and catechol.

The water-soluble electrophilic polymer that is present in the reactive polymer particles is preferably selected from polyoxazolines, polyethylene glycols, polyvinylpyrrolidones, polyurethanes (e.g. as described in WO 2017/171551) and combinations thereof. Even more preferably the electrophilic polymer is selected from polyoxazolines, polyethylene glycols and combinations thereof. Most preferably the electrophilic polymer is a polyoxazoline.

The polyoxazoline comprising reactive electrophilic groups is preferably derived from a polyoxazoline whose repeating units are represented by the following formula (I):

(CHR¹)ₘNCOR²

wherein R², and each of R¹ are independently selected from H, optionally substituted C₁₋₂₂ alkyl, optionally substituted cycloalkyl, optionally substituted aralkyl, optionally substituted aryl; and m being 2 or 3.

Preferably, R¹ and R² in formula (I) are selected from H and C₁₋₈ alkyl, even more preferably from H and C₁₋₄ alkyl. R¹ most preferably is H. The integer m in formula (I) is preferably equal to 2.

According to a preferred embodiment, the polyoxazoline is a polymer, even more preferably a homopolymer of 2-alkyl-2-oxazoline, said 2-alkyl-2-oxazoline being selected from 2-methyl-2-oxazoline, 2-ethyl-2-oxazoline, 2-propyl-2-oxazoline, 2-butyl-2-oxazoline and combinations thereof. Preferably, the polyoxazoline is a homopolymer of 2-propyl-2-oxazoline or 2-ethyl-oxazoline. Most preferably, the polyoxazoline is a homopolymer of 2-ethyl-oxazoline.

According to a particularly preferred embodiment, the electrophilic polymer carrying reactive electrophilic groups comprises at least 20 oxazoline units, more preferably at least 30 oxazoline units and most preferably at least 80 oxazoline units. The electrophilic polymer preferably comprises on average at least 0.05 reactive electrophilic groups per oxazoline residue. Even more preferably, the electrophilic polymer comprises on average at least 0.1 reactive electrophilic groups per oxazoline residue. Most preferably, the electrophilic polymer comprises on average 0.12-0.5 reactive electrophilic groups per oxazoline residue.

The electrophilic polymer typically carries on average at least 10, more preferably at least 20 reactive electrophilic groups.

Polyoxazoline can carry reactive electrophilic groups in its side chains (pendant reactive electrophilic groups), at its termini, or both. The polyoxazoline that is employed in accordance with the present invention advantageously contains one or more pendant reactive electrophilic groups. Typically, the polyoxazoline contains 0.03-0.5 pendant reactive electrophilic groups per monomer, more preferably 0.04-0.35 pendant reactive electrophilic groups per monomer, even more preferably 0.05-0.25 pendant reactive electrophilic groups per monomer.

The polyethylene glycol (PEG) comprising reactive electrophilic groups that is applied in accordance with the present invention preferably is a multi-arm PEG or a star PEG, comprising at least 3 arms, more preferably at least 4 arms terminated with a reactive electrophilic group.

The nucleophilic cross-linking agent in the reactive polymer particles typically contains at least 3 reactive nucleophilic groups, more preferably at least 5 reactive nucleophilic groups, even more preferably at least 10 reactive nucleophilic groups, most preferably at least 20 reactive nucleophilic groups.

The reactive nucleophilic groups of the nucleophilic cross-linking agent are preferably selected from amine groups, thiol groups, phosphine groups and combinations thereof. More preferably, these reactive nucleophilic groups are selected from amine groups, thiol groups and combinations thereof. Even more preferably, the reactive nucleophilic groups are amine groups. These amine groups are preferably selected from primary amine groups, secondary amine groups and combinations thereof. Most preferably, the amine groups are primary amine groups.

The nucleophilic cross-linking agent preferably has a nitrogen content of at least 1 wt.%, more preferably of 5-10 wt.% and most preferably of 15-25 wt.%.

In one embodiment of the invention the nucleophilic cross-linking agent is a low molecular weight polyamine having a molecular weight of less than 1,000 g/mol, more preferably of less than 700 g/mol and most preferably of less than 400 g/mol. Examples of suitable low molecular weight polyamines include dilysine; trilysine; tetralysine; pentalysine; dicysteine; tricysteine; tetracysteine; pentacystein; oligopeptides comprising two or more amino acid residues selected from lysine, ornithine, cysteine, arginine and combinations thereof, and other amino acid residues; spermine; tris(aminomethyl)amine; arginine and combinations thereof.

According to another embodiment of the invention, the nucleophilic cross-linking agent is a high molecular weight (> 1,000 g/mol) polyamine selected from the group of nucleophilically activated POx (NU-POx); amine-functionalised polyethylene glycol, chitosan; chitosan derivatives (e.g. dicarboxy-derivatised chitosan polymers as described in WO 2009/028965), polyethyleneimines; polyvinylamine; polyallyl amine; amine-functionalized poly(meth)acrylates; saccharides containing amine-functional moieties such as aminoglycosides; polypeptides comprising two or more amino acid residues selected from lysine, ornithine, cysteine, arginine and combinations thereof; and combinations thereof. Albumin of natural source or recombinant is an example of a polypeptide that may suitably be employed as a polypeptide. 4,6-disubstituted deoxystreptamine (Kanamycin A, Amikacin, Tobramycin, Dibekacin, Gentamicin, Sisomicin, Netilmicin), 4, 5 -di substituted deoxystreptamine (Neomycins B, C and Neomycin E (paromomycin)) and nondeoxystreptamine aminoglycosides, e.g. streptomycin, are examples of aminoglycosides that can be employed. Most preferably, the high molecular weight polyamine is NU-POx.

In yet another embodiment of the invention the nucleophilic cross-linking agent employed in the cross-linked polymer is a low molecular weight polythiol comprising 2 or more thiol groups having a molecular weight of less than 1,000 g/mol, more preferably of less than 700 g/mol and most preferably of less than 400 g/mol. Examples of suitable low molecular weight polyamines include trimercaptopropane, ethanedithiol, propanedithiol, 2-mercaptoethyl ether, 2,2'-(ethylenedioxy)diethanethiol, tetra(ethylene glycol) dithiol, penta(ethylene glycol) dithiol, hexaethylene glycol dithiol; thiol modified pentaerythritol, dipentaerythritol, trimethylolpropane or ditrimethylolpropane; oligopeptides containing at least two cysteine residues.

According to yet another embodiment of the invention, the nucleophilic cross-linking agent employed in the cross-linked polymer is a high molecular weight (> 1,000 g/mol) polythiol selected from the group of: NU-POx comprising at least two thiol groups; thiol-functionalized poly(meth)acrylates; polysaccharides containing thiol-functional moieties, polypeptides comprising two or more cysteine residues.

Preferably, the nucleophilic cross-linking agent is a high molecular weight polyamine selected from the group of nucleophilically activated POx (NU-POx); amine-functionalised polyethylene glycol, chitosan; chitosan derivatives, polyethyleneimines; polyvinylamine; polyallyl amine; amine-functionalized poly(meth)acrylates; saccharides containing amine-functional moieties; polypeptides comprising two or more amino acid residues selected from lysine, ornithine, cysteine, arginine and combinations thereof; and combinations thereof. More preferably, the nucleophilic cross-linking agent is selected from NU-POx; amine-functionalised polyethylene glycol; gelatin, collagen, chitosan and combinations thereof.

Chitosan is a biodegradable, nontoxic, complex carbohydrate derivative of chitin (poly-N-acetyl-D-glucosamine), a naturally occurring substance. Chitosan is the deacetylated form of chitin. The chitosan applied in accordance with the present invention preferably has a degree of deacetylation of more than 70%.

The high molecular weight polyamine preferably comprise three or more amine groups, more preferably 10 or more amine groups, and most preferably 20 or more amine groups.

According to a particularly preferred embodiment, the nucleophilic cross-linking agent is selected from gelatin, collagen, chitosan and combinations thereof. Even more preferably the nucleophilic cross-linking agent is selected from gelatin, collagen and combinations thereof, gelatin being most preferred. Gelatin is preferably applied in the reactive polymer particles in the form of cross-linked gelatin, most preferably in the form of reduced cross-linked gelatin. Reduced cross-linked gelatin is a cross-linked gelatin (gelfoam) that has been partially hydrolysed. Partial hydrolysis of the peptide bonds in the cross-linked gelatin can be achieved by e.g. alkaline treatment. Hydrolysis of the cross-linked gelatin results in an increased density of free carboxyl and free amine groups and in increased water solubility.

According to a preferred embodiment, the nucleophilic cross-linking agent contains two or more reactive amine groups and the reactive electrophilic groups of the electrophilic polymer are selected from carboxylic acid esters, sulfonate esters, phosphonate esters, pentafluorophenyl esters, p-nitrophenyl esters, p-nitrothiophenyl esters, acid halide groups, anhydrides, ketones, aldehydes, isocyanato, thioisocyanato, isocyano, epoxides, activated hydroxyl groups, glycidyl ethers, carboxyl, succinimidyl esters, sulfosuccinimidyl esters,, imido esters, dihydroxy-phenyl derivatives, and combinations thereof.

According to another preferred embodiment, the nucleophilic cross-linking agent contains two or more reactive thiol groups and the reactive electrophilic groups of the electrophilic polymer are selected from halo acetals, orthopyridyl disulfide, maleimides, vinyl sulfone, dihydroxyphenyl derivatives, vinyl, acrylate, acrylamide, iodoacetamide, succinimidyl esters, sulfosuccinmidyl esters and combinations thereof. More preferably, the reactive electrophilic groups are selected from succinimidyl esters, sulfosuccinimidyl esters, halo acetals, maleimides, or dihydroxyphenyl derivatives and combinations thereof. Most preferably, the reactive electrophilic groups are selected from maleimides or dihydroxyphenyl derivatives and combinations thereof.

Besides the water-soluble electrophilic polymer and the nucleophilic cross-linking agent, the reactive polymer particles may suitably contain a polysaccharide selected from dextran, alginate, oxidized cellulose (including oxidized regenerated cellulose (ORC)), hydroxyethylcellulose, hydroxymethylcellulose, hyaluronic acid; and combinations thereof. Preferably, such a polysaccharide is contained in the reactive polymer particles in a concentration of at least 15 wt.%, more preferably of at least 25 wt.% and most preferably of at least 50 wt.%.

The reactive polymer particles containing the water-soluble electrophilic polymer and the nucleophilic cross-linking agent can be produced via wet granulation and subsequent drying, preferably under reduced pressure. The granulation liquid should be chosen such that little or no reactions occur during granulation between the electrophilic polymer and the nucleophilic cross-linking agent. This may be achieved, for instance, by employing a granulation liquid in which at least one of these two components is insoluble. Most preferably, the water-soluble polymer carrying reactive electrophilic groups is insoluble in the granulation liquid.

According to a particularly preferred embodiment, the reactive polymer particles are particle agglomerates comprising: (i) electrophilic particles containing the water-soluble electrophilic polymer; and (ii) nucleophilic particles containing the nucleophilic cross-linking agent.

The electrophilic particles preferably contain at least 30 wt.%, more preferably at least 50 wt.% and most preferably at least 80 wt.% of the water-soluble electrophilic polymer.

The nucleophilic particles preferably contain at least 30 wt.%, more preferably at least 50 wt.% and most preferably at least 80 wt.% of the nucleophilic cross-linking agent.

Typically, the reactive hybrid particles according to this embodiment have the following composition:
(a) 25-75 wt.% of the water-soluble electrophilic polymer;
(b) 25-75wt.% of the nucleophilic cross-linking agent;
(c) 0-50 wt.% polysaccharide;
wherein the combination of components (a) to (c) together constitute at least 80 wt.%, more preferably at least 90 wt.% of the reactive polymer particles. According to a particularly preferred embodiment, the electrophilic polymer that is employed in this embodiment is a polyoxazoline (EL-POx).

The reactive polymer particles preferably contain water-soluble electrophilic polymer and the nucleophilic cross-linking agent in such amounts that the ratio between the total number of reactive electrophilic groups provided by the water-soluble electrophilic polymer and the total number of reactive nucleophilic groups provided by the nucleophilic cross-linking agent lies in the range of 1:1.5 to 1.5:1, more preferably in the range of 1:1.2 to 1.2:1 and most preferably in the range of 1:1.1 to 1.1:1.

In accordance with another advantageous embodiment the reactive polymer particles contain a dry buffering system. Preferably, the buffering system has a buffering pH in the range of 7 to 11, more preferably in the range of 8 to 10. Preferably, the buffering system has a buffer capacity of at least 10 mmol.l⁻¹.pH⁻¹. More preferably, the buffer capacity is a least 25 mmol.l^{- 1}.pH⁻¹, most preferably the buffer capacity is at least 50 mmol.l⁻¹.pH⁻¹

According to a particularly preferred embodiment of the present invention, the fibrous carrier structure comprises fibres that contain oxidized cellulose and the reactive polymer particles contain EL-POx in combination with a nucleophilic cross-linking agent. Preferably the nucleophilic cross-linking agent is selected from NU-POx; amine-functionalised polyethylene glycol; gelatin; collagen and combinations thereof.

In another preferred embodiment, the reactive polymer particles contain at least 25 wt.%, most preferably at least 50 wt.% of gelatin, preferably in the form of reduced crosslinked gelatin.

The inventors have unexpectedly discovered that a haemostatic sheet having particularly desirable adhesive properties can obtain by employing reactive polymer particles containing 1-20 wt.%, more preferably 2.5-15 wt.% and most preferably 5-10 wt.% of a non-reactive non-ionic polymer. This non-reactive non-ionic polymer does not contain reactive electrophilic groups or reactive nucleophilic groups.

In a very preferred embodiment, the reactive polymer particles are coated with the non-reactive non-ionic polymer.

The non-reactive non-ionic polymer preferably has a melting point in the range of 40-70°C, more preferably in the range of 45-65°C and most preferably in the range of 50-60°C. Here the melting point refers to the temperature at which the polymer is completely melted.

Examples of non-reactive non-ionic polymers that may suitably be applied in the reactive polymer particles of the present invention include poloxamers, polyethylene glycols and combinations thereof. Poloxamer is a non-ionic triblock copolymer composed of a central hydrophobic chain of polyoxypropylene (polypropylene oxide)) flanked by two hydrophilic chains of polyoxyethylene (poly(ethylene oxide)) and is represented by formula (I) 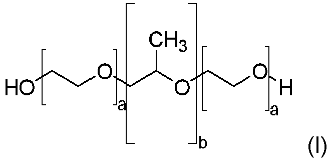 wherein a is an integer of from 10 to 110 and b is an integer of from 20 to 60. When a is 80 and b is 27, this polymer is known as poloxamer 188. Other known poloxamers useful in the present invention are poloxamer 237 (a = 64; and b = 37), poloxamer 338 (a = 141; and b = 44) and poloxamer 407 (a = 101; and b = 56). Further poloxamers that are known and can be useful in the present invention include poloxamer 108, poloxamer 182, poloxamer 183, poloxamer 212, poloxamer 217, poloxamer 238, poloxamer 288, poloxamer 331, poloxamer 338 and poloxamer 335.

According to a particularly preferred embodiment, the non-reactive non-ionic polymer is a poloxamer, even more preferably a poloxamer having an average molecular mass of 2,000-18,000, most preferably a poloxamer having an average molecular mass of 7,000-10,000.

The poloxamer applied in the particle agglomerates preferably is a solid at room temperature.

Another aspect of the present invention relates to a sealed package containing one or more haemostatic sheets according to the present invention.

Yet another aspect of the invention relates to a method of preparing a haemostatic sheet, said method comprising:
- providing a sheet of cohesive fibrous carrier structure as defined above;
- providing reactive polymer particles as defined above; and
- distributing the reactive polymer particles within the interstitial space of the fibrous carrier structure.

The reactive polymer particles may be distributed within the interstitial space using a dry method or a wet method, the dry method being preferred. In the dry method, the reactive polymer particles are applied in the form of a powder and this powder is dispersed in dry form through the interstitial space.

According to one preferred embodiment of this dry method, the reactive polymer particles are distributed within the interstitial space of the fibrous carrier structure by shaking or vibrating the sheet of fibrous carrier structure.

In accordance with another embodiment of the dry method, the haemostatic sheet is prepared by a lamination method comprising:
a) providing a sheet of fibrous carrier structure,
b) depositing a layer of the reactive polymer particles onto the sheet of fibrous carrier structure;
c) superimposing another sheet of fibrous carrier structure onto the layer of reactive polymer particles; and
optionally repeating steps b) and c) one or more times.

Distribution of the reactive polymer particles within the laminate so obtained may be promoted by shaking or vibration the laminate.

In the wet method of distributing reactive polymer particles, a dispersion of the reactive polymer particles in a low boiling organic liquid is used to impregnate the fibrous carrier structure, followed by evaporation of the low boiling organic liquid, preferably at reduced pressure. Typically, the low boiling organic liquid has a boiling point at atmospheric pressure of less than 150°C, more preferably of less 98°C and most preferably of less than 80°C.

Preferably, in the present method the carrier structure containing the distributed reactive polymer particles is heated to a temperature above the glass temperature of the water-soluble electrophilic polymer for at least 5 minutes. By heating the reactive polymer particles in this way, the particles will become sticky and adhere to the fibrous carrier structure. Typically, the polymer particles are heated to a temperature of at least 40°C, more preferably of 50-80°C for at least 15 minutes.

The present method preferably comprises sterilisation of the haemostatic sheet. The haemostatic sheet may be sterilised prior to aseptic packaging. Alternatively, the sheet may be sterilised within a sealed packaging.

The invention is further illustrated by the following non-limiting examples.

### EXAMPLES

Haemostatic sheets not comprising a cohesive fibrous carrier structure comprising a three-dimensional interconnected interstitial space and a plurality of reactive polymer particles comprising (i) and (ii) distributed within the interstitial space as defined in claim 1 are not part of the claimed invention.

In general: wherever residual moisture content (in dried powder, granulate and/or cohesive fibrous carrier structures) after drying is not explicitly mentioned, levels are below 2.0% w/w.

### Preparation of NHS-POx

NHS-side chain activated poly[2-(ethyl/hydroxy-ethyl-amide-ethyl/NHS-ester-ethyl-ester-ethyl-amide-ethyl)-2-oxazoline] terpolymer containing 20% NHS-ester groups (= EL-POx, 20% NHS) was synthesized as follows:
Poly[2-(ethyl/methoxy-carbonyl-ethyl)-2-oxazoline] copolymer (DP = +/-100) was synthesized by means of CROP using 60% 2-ethyl-2-oxazoline (EtOx) and 40% 2-methoxycarbonyl-ethyl-2-oxazoline (MestOx). A statistical copolymer containing 40% 2-methoxycarbonyl-ethyl groups (1H-NMR) was obtained. Secondly, the polymer containing 40% 2-methoxycarbonyl-ethyl groups, was reacted with ethanolamine yielding a copolymer with 40% 2-hydroxy-ethyl-amide-ethyl-groups (1H-NMR). After that, half of the 2-hydroxy-ethyl-amide-ethyl-groups was reacted with succinic anhydride yielding a terpolymer with 60% 2-ethyl groups, 20% 2-hydroxy-ethyl-amide-ethyl-groups and 20% 2-carboxy-ethyl-ester-ethyl-amide-ethyl-groups according to 1H-NMR. Lastly, the 2-carboxy-ethyl-ester-ethyl-amide-ethyl-groups were activated by N-hydroxysuccinimide (NHS) and diisopropylcarbodiimide (DIC), yielding EL-POx, 20% NHS. The NHS-POx contained 20% NHS-ester groups according to ¹H-NMR. NHS-POx was dissolved between 2-8 °C in water (60 g in 300 mL), cooled at minus 80 °C for half an hour and freeze dried. The freeze dried powder so obtained was dried in a Rotavap at 40 °C until the water content was below 0.8% w/w as determined via Karl Fischer titration. This dry (white) powder was grinded using a ball mill (Retch MM400) until the average particle size was not more than 40 µm (D [4,3]) and vacuum sealed in alu-alu bags.

### Dying of NHS-POx powder

20 grams of NHS-POx powder was dissolved in water and mixed with 50 mg Brilliant Blue R (Sigma Aldrich) using a high-performance dispersing instrument (Ultra-Turrax, IKA). Directly after mixing (2 minutes) the solution was frozen at -78 °C and subsequently freeze dried overnight. The freeze dried powder so obtained was dried in a Rotavap at 40 °C until the residual water content was below 0.8% w/w as determined via Karl Fischer titration. Next, the dried (blue) powder was grinded using a ball mill (Retch MM400) until a blue dyed NHS-POx powder having an average particle size of not more than 40 µm (D [4,3]) and vacuum sealed in alu-alu bags.

### Preparation of NU-POx

Polyoxazoline containing ethyl and amine groups in the alkyl side chain was synthesized by CROP of EtOx and MestOx and subsequent amidation of the methyl ester side chains with ethylene diamine to yield a poly(2-ethyl/aminoethylamidoethyl-2-oxazoline) copolymer (NU-POx). The NU-POx contained 10% NH₂ according to ¹H-NMR. NU-POx was dissolved between 2-8 °C in water (60 g in 300 mL), cooled at minus 80 °C for half an hour an freeze dried. The freeze dried powder so obtained was dried in a Rotavap at 40 °C until the water content was below 0.8% w/w as determined via Karl Fischer titration. This dry powder was grinded in a table top grinding machine until the average particle size was not more than 100 µm (D [4,3]) and vacuum sealed in alu-alu bags.

### Preparation of reactive NHS-POx / NU-POx granules

Blue or white (non-dyed) NHS-POx powder was wetted with isopropyl alcohol (IPA) in a high shear mixer until a homogeneous snow-like powder was obtained containing about 1-2% w/w IPA. After this, NU-POx powder was added and mixed. The wetted blue NHS-POx powder was mixed with NU-POx powder in a molar ratio of 1:0.6, said molar ratio referring to the ratio of the number of NHS groups provided by NHS-POx to the number of amine groups provided by the NU-POx. The wetted non-dyed NHS-POx powder was also mixed with NU-POx powder in other molar ratios (1:0.8; 1:1 and 1:1.2).

After mixing, the wet granulates were dried under reduced pressure until the IPA content was less than 0.1% w/w as determined via ¹H-NMR. The dried granulates were grinded using a ball mill (Retch MM400) until the average particle size was not more than 50 µm (D [4,3]) and vacuum sealed in alu-alu bags.

The particle size distribution of the granulates so obtained was approximately was: 90 vol.% < 20 µm, 70 vol.% < 10 µm and 40 vol.% < 5 µm.

The NHS-POX/NU-POx granulate (1:1) was analysed using ¹H-NMR spectroscopy. 25 mg of granulate were dissolved in trifluoroacetic acid (0.20 mL) by sonicating for 20 minutes. After complete dissolution of the granulate, the sample was diluted with deuterated dimethylsulfoxide (DMSO-*d₆*) containing maleic acid (2.5 mg/mL) as an internal standard (0.80 mL), transferred to an NMR tube and a ¹H-NMR spectrum was recorded. From the acquired spectrum, the amount of NHS bound to NHS-POx can be calculated, along with the molar ratio of NHS and amine groups present in the granulate. The amount of NHS bound to NHS-POx in the granulate was equal to the amount of NHS bound to NHS-POx starting material indicating no decay or cross linking during granulation.

The total polymer recovery, *i.e.* the combination of NHS-POx and NU-POx, in the NMR sample was determined using a known amount of internal standard (maleic acid) and a calibration curve constructed from ¹H-NMR spectra recorded of NHS-POx and NU-POx in different concentrations. The total polymer recovery was measured to be 99 percent, indicating that no insoluble crosslinked material was formed.

The NHS-POX/NU-POx granulate (1:1) was further analysed by means of size exclusion chromatography. 20 mg of the granulate was treated with acetic anhydride (1.00 mL) for 1 hour at 50°C. Subsequently, methanol (2.00 mL) was added and the mixture was stirred for an additional hour at 50°C. An aliquot (0.75 mL) was taken and all volatiles were removed under reduced pressure. The sample was taken up in *N*,*N*-dimethylacetamide containing 50 mM lithium chloride (2.50 mL), which was the eluent for SEC analysis. SEC was measured against poly(methyl methacrylate) standards and from the obtained size exclusion chromatogram, the Mₙ, M_{w} and PDI were determined. The PDI was not more than 1.5, indicating no cross linking had occurred during granulation. Analytical validation of this size exclusion chromatographic method indicated that intentional cross linking of NHS-POx with NU-POx at a level of 0.05 mol% increased the PDI to more than 2.5.

### Preparation of reactive NHS-POx / NU-POx / P188 granules

Reactive NHS-POx / NU-POx granules was prepared as described previously. Subsequently, P188 coated reactive NHS-POx / NU-POx granulate (2.5% w/w or 5% w/w P188) was prepared by heating the NHS-POx / NU-POx granulate together with P188 powder in a high shear mixer at 65 °C for 10 minutes followed by cooling down to ambient conditions. The coated granulate was grinded using a ball mill (Retch MM400) until the average particle size was not more than 40 µm (D [4,3]) and vacuum sealed in alu-alu bags.

The particle size distribution of the granulates so obtained was approximately: 90 vol.% < 80 µm, 50 vol.% < 40 µm and 10 vol.% < 10 µm.

The NHS-POx/NU-POx/P188 (2.5%) granulate was analysed using ¹H-NMR spectroscopy. 25 mg of powder were dissolved in trifluoroacetic acid (0.20 mL) by sonicating for 20 minutes. After complete dissolution of the granulate, the sample was diluted with deuterated dimethylsulfoxide (DMSO-*d₆*) (0.80 mL), transferred to an NMR tube and a ¹H-NMR spectrum was recorded. From the obtained spectrum, the amount of non-reacted NHS was calculated to be 98 percent compared to NHS-POx.

The NHS-POx/NU-POx/P188 (2.5%) granulate was further analysed by means of size exclusion chromatography. 20 mg of the granulate was treated with acetic anhydride (1.00 mL) for 1 hour at 50°C. Subsequently, methanol (2.00 mL) was added and the mixture was stirred for an additional hour at 50°C. An aliquot (0.75 mL) was taken and all volatiles were removed under reduced pressure. The sample was taken up in *N*,*N*-dimethylacetamide containing 50 mM lithium chloride (2.50 mL), which was the eluent for SEC analysis. SEC was measured against poly(methyl methacrylate) standards and from the obtained size exclusion chromatogram, the Mₙ, M_{w} and PDI were determined. The PDI was not more than 1.5, indicating that no cross linking had occurred during granulation.

### Preparation of reduced crosslinked gelatin (RXL)

Reduced crosslinked gelatin (RXL) was prepared according to three procedures:
- 12 g of gelatin powder (Gelita-SPONO, Gelita Medical GmbH) were dissolved in 350 mL of a 0.1 molar aqueous sodium hydroxide solution by stirring for 2 hours at 40°C. After a clear solution was obtained, the mixture was allowed to cool down to ambient temperature and the pH was adjusted to 7 by addition of 32.5 mL of a 1.0 molar aqueous hydrochloric acid solution. The solution was flash frozen using liquid nitrogen and freeze dried. Subsequently, the powder was milled in a coffee grinder, dried under reduced pressure and vacuum sealed in an alu-alu bag. Hereafter, this reduced crosslinked gelatin will be referred to as RXL-LS (low salt).
- 12 g of gelatin powder (Gelita-SPONO, Gelita Medical GmbH) were dissolved in 360 mL of a 1.0 molar aqueous sodium hydroxide solution by stirring for 10 minutes at 40°C. The obtained clear solution was cooled to ambient temperature and the pH was decreased to 7 by addition of 30 mL of a concentrated hydrochloric acid solution (37 % w/w). The solution was flash frozen using liquid nitrogen and freeze dried. Subsequently, the powder was milled in a coffee grinder, dried under reduced pressure and vacuum sealed in an alu-alu bag. Hereafter, this reduced crosslinked gelatin will be referred to as RXL-HS (high salt).

### Preparation of reactive NHS-POx / RXL granules (low salt and high salt)

NHS-POx/RXL reactive granules were prepared as follows: 5 g of blue NHS-POx powder were wetted with IPA in a high shear mixer until a homogeneous snow like powder was obtained containing about 1-2% w/w IPA. After this, 5 g of RXL, RXL-LS or RXL-HS powder were added and mixed. After mixing, the wet granulates were dried under reduced pressure until the IPA content was less than 0.1% w/w as determined via ¹H-NMR. The dried granulates were milled in a coffee grinder until the average particle size was not more than 90 µm (D [4,3]) and vacuum sealed in alu-alu bags.

The particle size distribution of the granulates so obtained was approximately: 90 vol.% < 190 µm, 50 vol.% < 60 µm and 10 vol.% < 15 µm.

The granulate containing RXL was analysed by means of ¹H-NMR spectroscopy analysis. To this end deuterated chloroform (CDCl3) containing 5 %(v/v) acetic acid (1.0 mL) was added to 25 mg of the granulate. NHS-POx was selectively extracted by sonicating the sample for 20 minutes. The dispersion was passed through a 0.22 µm filter, transferred to an NMR tube and a ¹H-NMR spectrum was recorded. From the obtained spectrum, the amount of non-reacted NHS was calculated to be 98 percent compared to NHS-POx.

The recovery of NHS-POx in the NMR sample was determined using trimethylsilane as an internal standard and a calibration curve constructed from ¹H-NMR spectra of NHS-POx in different concentrations. The total NHS-POx recovery was measured to be 100 percent, indicating that no insoluble crosslinked material was formed.

The NHS-POx/RXL granulate was further analysed by means of size exclusion chromatography. Therefore, an aliquot (0.15 mL) was taken from the solution used for ¹H-NMR spectroscopy analysis. This solution was diluted with *N*,*N*-dimethylacetamide containing 50 mM lithium chloride (1.00 mL), which was the eluent for SEC analysis. SEC was measured against poly(methyl methacrylate) standards and from the obtained size exclusion chromatogram, the Mₙ, M_{w} and PDI were determined. The PDI was not more than 1.5, again indicating that no cross linking had occurred during granulation.

### Preparation of reactive NHS-POx / RXL granules containing carbonate

First, a 1:1 mole/mole mixture of sodium carbonate and sodium hydrogen carbonate was prepared by dissolving 25.31 g of sodium carbonate and 20.06 g of sodium hydrogen carbonate in 350 mL ultrapure water. The solution was flash frozen in liquid nitrogen and freeze dried. The resulting powder was dried under reduced pressure and vacuum sealed in an alu-alu bag.

The NHS-POx/RXL/carbonate granulates were prepared as follows: 5 g of RXL-LS or RXL-HS and 0.178 g of the sodium carbonate / sodium hydrogen carbonate were mixed using a high shear mixer. Next, 5 g of blue NHS-POx were added containing about 1-2% w/w IPA and mixed until a homogeneous powder was obtained. After mixing, the wet granulates were dried under reduced pressure until the IPA content was less than 0.1% w/w as determined via ¹H-NMR. The dried granulates were milled in a coffee grinder until the average particle size was not more than 100 µm (D [4,3]) and vacuum sealed in alu-alu bags.

### Preparation of reactive NHS-PEG / RXL (low salt) granules

0.62 grams of RXL-LS was mixed with 0.88 grams of NHS-PEG 4-arm MW 10k (ex Creative PEGWorks) in a pestle and mortar until a homogeneous powder was obtained; about 2 drops of IPA were added and mixed. After mixing, the granulates were dried under reduced pressure and vacuum sealed in alu-alu bags.

### Preparation of reactive NHS-PEG / RXL (low salt) granules containing carbonate

0.62 grams of RXL-LS was mixed with 0.12 grams of a freeze dried 1:1 mole/mole mixture of sodium carbonate and sodium hydrogen carbonate in a pestle and mortar until a homogeneous powder was obtained. Next, 0.84 gram of NHS-PEG 4-arm MW 10k (ex Creative PEGWorks) was mixed with the RXL / carbonate powder; about 2 drops of IPA were added and mixed. After obtaining a homogeneous granulate, it was dried under reduced pressure and vacuum sealed in an alu-alu bag.

### Cohesive fibrous carrier structures

The following commercially available haemostatic products were selected to be used as fibrous carrier structures in the preparation of haemostatic sheets according to the present invention:
- SURGICEL^{®} SNoW^{™}: a cohesive fibrous carrier structure consisting of absorbable nonwoven fabric of fibres prepared by the controlled oxidation of regenerated cellulose (ORC). Dimensions are 5.1 cm × 10.2 cm and the water content was not more than 2.0% w/w
- SURGICEL^{®} NU-KNIT^{®}/ SURGICEL^{®} SNoW^{™}-Hybrid: a densely knit absorbable fabric of fibres prepared by the controlled oxidation of regenerated cellulose (ORC), available in several sizes (e.g., 15.2cm × 22.9cm) knitted onto SURGICEL^{®} SNoW^{™} cut to a dimension of 5.1 cm × 10.2 cm.
- A cohesive fibrous carrier structure consisting of oxidized regenerated cellulose (ORC) and polyglactin 910 having dimensions (length, width) of 97.0 to 102.5 mm and a thickness of 1.40 to 2.50 mm was prepared as follows. Poly (glycolide-co-lactide) (PGL, 90/10 mol/mol) was melt-spun into fiber. A 80 denier multifilament yarn was consolidated into a 800 denier consolidated yarn. The consolidated yarn was crimped at approximately 110° C. The crimped yarn was cut into staple having a length of about 1.25". 20 g of the crimped staple was accurately weighed and laid out uniformly on the feed conveyor belt of a multi-roller carding machine. The environmental conditions (temp: 21° C./55% RH) were controlled. The staple was then carded to create a nonwoven batt. The batt was removed from the pick-up roller and cut into 4 equal parts. These were re-fed into the carder perpendicular to the collection direction. After this second pass the batt was weighed (19.8 g: 99% fabric yield) and then compacted into a felt. The compact felt was precisely laid onto an ORC fabric and firmly attached via 2 passes in the needlepunching equipment. The multilayered fabric was trimmed and scoured in 3 discrete isopropyl alcohol baths to remove spin finish and any machine oils. The scoured multilayered fabric was dried in an oven at 70° C. for 30 minutes, cooled and weighed. The water content was not more than 2.0% w/w

### Bleeding experiments

Standardized *ex-vivo* and *in-vivo* porcine bleeding models were used to assess haemostatic efficacy. All models use heparin to increase clotting time of blood to about 2 to 3 times activated coagulation time (ACT).

*Ex-vivo* model: live *ex-vivo* pig model with a fresh liver, perfused with heparinized fresh blood from the slaughterhouse to mimic real *in-vivo* situations a closely as possible. Livers are mounted onto a perfusion machine by which oxygenation, pH of blood, temperature and blood pressure are kept within *vivo* boundaries. Two livers and 10 litres of heparinized blood (5000 units/L) are collected at the slaughterhouse. Livers are transported on ice; blood at ambient temperature. Within two hours after collection, livers are inspected for lesions which are closed with gloves and cyanoacrylate glue.
- Perfusion parameters: flow 600 ml/min; pressure 10-12 mmHg; temperature 37 °C (+/- 1 °C); carbogen 0.25 litres a minute
- With a flat, round, rotating abrasion tool a circular bleeding wound (8 mm diameter) is created on the liver surface, with a rubber onlay so that the depth of the punched bleeding is always 3 mm
- After the liver is perfused properly (colour and temperature checked) samples are tested according to the following procedure: cut sample to the right size (2.7 by 2.7 cm); camera on; site number on camera; biopsy punch 8 mm; cut away biopt; remove blood from bleeding with gauze (2x); collect blood for 30 sec in pre-weight gauze; score bleeding (by 2 researchers); put sample on bleeding by a pre-wetted gauze (saline) and hold with little pressure for 1 min; observe for 5 min (check and score adhesion and haemostasis) and repeat after 30 minutes.

*In-vivo* model: standardized combined penetrating spleen rupture is inflicted in anesthetized swine (Domestic Pig, Male, Body Weight Range: 40 kg, Adult).

A midline laparotomy is performed to access the spleen and other organs. Using a scalpel, n=3 (S1... S3) subcapsular standardized lesions (10 mm × 10 mm) are made. The haemostatic products are applied with gentle pressure by a pre-wetted gauze (saline) and held for 1 min. After application of the product the time to haemostasis (TTH) is assessed. If TTH equals zero, this means that after 1 minute pressure haemostasis had already been achieved.

### Scoring system for patches: Coagulation

| | |
|---|---|
| ++++ | Achieved immediately after tamponade |
| +++ | Achieved <10 seconds after tamponade |
| ++ | Achieved <30 seconds after tamponade |
| + | Achieved within 3 minutes after tamponade |
| +/- | Achieved after 3 minutes, second tamponade applied |
| - | Not achieved |

### Scoring system for patches: Adhesion 10 minutes after application

| | |
|---|---|
| ++++ | Very strong adhesion (patch breaks when being removed) |
| +++ | Strong adhesion (patch breaks when being removed) |
| ++ | Strong adhesion (patch can be removed without breaking) |
| + | Moderate adhesion (patch can be removed without breaking) |
| +/- | Mild adhesion (patch can be removed without breaking) |
| - | Not achieved |

### Example 1

### Impregnation of carrier structures with dyed polymer particles

A Vibratory Sieve Shaker AS 300 (Retsch) was operated for two consecutive periods of 5 minutes each to introduce the dyed NHS-POx powder into different carrier structures. After production, the haemostatic patches were packed in alu-alu pouches containing 1 g of silica and vacuum sealed.

Using the above mentioned shaking machine, blue NHS-POx powder was introduced into three different carrier structures:
- gelatin foam (Gelita Rapid^{®}, Gelita Medical Germany), a sponge
- collagen foam (Surgicoll^{®}, MBP, Germany), a sponge
- fibrous carrier structure (SURGICEL^{®} SNoW^{™}, Ethicon, USA)

It was found that after the shaking treatment, the blue NHS-POx powder had hardly penetrated the gelatin foam or collagen foam. After cutting the foam with a scalpel it was clear that there was no depth impregnation. In contrast thereto, the shaking treatment had homogenously dispersed the blue NHS-POx powder throughout the fibrous carrier structure.

### Example 2

Hemostatic patches (SURGICEL^{®} SNoW^{™}; 5.1 x10.2 cm, appr. 0.37 grams) were impregnated with the reactive NHS-POx/NU-POx granulates. The molar ratio of NHS-POx to NU-POx in these granulates varied from 1:0.6 to 1:2, said molar ratio referring to the ratio of the number of NHS groups provided by NHS-POx to the number of amine groups provided by the NU-POx.

One gram of granulate was distributed throughout the patches by shaking them in Vibratory Sieve Shaker AS 300 (Retsch) for two consecutive periods of 5 minutes each. Next, the hemostatic patches were packed in alu-alu pouches containing 1 g of silica and vacuum sealed.

The patches were cut into 2x2 cm pieces and tested (n=3) in the ex *vivo* liver perfused model. Time to hemostasis after 1-minute pressure (TTH), hemostatic capacity as well as adhesion were determined. The best performing patch (1:1 NHS-POx:NU-POx) was also tested in the *in vivo* porcine heparinized model on both liver and spleen resections. Results are shown in Table 1.

**Table 1**

| **NHS-POx** : **NU-POx** | ***Ex-vivo*** | | | ***In vivo*** | |
|---|---|---|---|---|---|
| | **TTH** | **Hemostatic capacity** | **Adhesive properties** | **Hemostatic capacity** | **Adhesive properties** |
| 1:0.6 | >2 min | +/- | +/- | NA | NA |
| 1:0.8 | >2 min | +/- | + | NA | NA |
| 1:1 | 0 | +++ | ++ | +++ | +++ |
| 1:2 | >2 min | +/- | +/- | NA | NA |

### Example 3

Cohesive fibrous carrier structures consisting of oxidized regenerated cellulose (ORC) and polyglactin 910 (appr. 0.72 grams), described herein before, were impregnated with the reactive NHS-POx/NU-POx (1:1) granulate or with NHS-POx/NU-POx (1:1) granulate coated with 5% w/w P188.

One gram of granulate was distributed throughout the patches by shaking them in Vibratory Sieve Shaker AS 300 (Retsch) for two consecutive periods of 5 minutes each. Next, the hemostatic patches were packed in alu-alu pouches containing 1 g of silica and vacuum sealed.

Patches were cut into 2.5x2.5 cm pieces and tested on standardized spleen and liver abrasions using the porcine *in vivo* model. The spleen and liver were externalized as the testing period progressed. Both organs were kept moist with saline soaked sponges or pad and/or saline lavage. Appropriately sized sections, ~4 cm², of the liver parenchyma/spleen were abraded to cause moderate to severe bleeding. The abrasions were created by a surgical scalpel. Patches were placed in direct contact with the bleeding area and manual tamponade was applied during 1 minute. Time to hemostasis after 1-minute pressure (TTH), hemostatic capacity as well as adhesion were determined. The results are shown in Table 2.

**Table 2**

| **Organ** | ***In-vivo* porcine model** | |
|---|---|---|
| | **Hemostatic capacity** | **Adhesive properties** |
| Liver * | +++ | + |
| Spleen * | +++ | + |
| Spleen ** | ++++ | ++++ |

| | | |
|---|---|---|
| *: granulate without P188 **: granulate incl. P188 | | |

### Example 4

Two different cohesive fibrous carrier structures described herein before were impregnated with different reactive polymer powders. The haemostatic properties of the patches so obtained were tested in the *ex-vivo* and *in-vivo* porcine bleeding models described herein before.

The fibrous carrier structures used were:

| | |
|---|---|
| • SW: | SURGICEL^{®} SNoW |
| • NS: | SURGICEL^{®} NU-KNIT^{®}/ SURGICEL^{®} SNoW^{™}-Hybrid |

These fibrous carrier structures were impregnated with 1.4 gram of powder using a pneumatic shaking device. The sheet of fibrous carrier was vibrated vertically. The engine of the long stroke type (NTK 25 AL L, ex Netter Vibration GmbH) was operated at 6 bar, 11 Hz and an amplitude of 30 mm. Four cycles of 15 seconds were used to disperse the powder into the fibrous carrier structure. The granulates were distributed through the complete thickness of the sheets. Also the distribution over the surface of the sheets was homogeneous.

Seven different reactive polymer granulates were tested. These granulates contained a water-soluble electrophilic polymer and a nucleophilic cross-linking agent. The preparation of these granulates has been described herein before.

The granulates that were tested are listed below:
- NHS-POx / NU-POx
- NHS-POx / NU-POx containing P188 (2.5%)
- NHS-POx / NU-POx containing carbonate and P188 (2.5%)
- NHS-POx / RXL (high salt)
- NHS-POx / RXL (high salt) containing carbonate
- NHS-POx / RXL (low salt)
- NHS-POx / RXL (low salt) containing carbonate

The different combinations of fibrous carrier structure and reactive polymer powders that were tested are shown in Table 3.

**Table 3**

| **Patch** | **Carrier structure** | **Electrophilic polymer** | **Nucleophilic cross-linking agent** | **Carbonate** | **P188 coating** |
|---|---|---|---|---|---|
| 1 | SW | NHS-POx | NU-POx | | |
| 2 | SW | NHS-POx | RXL-HS | | |
| 3 | SW | NHS-POx | RXL-HS | X | |
| 4 | SW | NHS-POx | RXL-LS | X | |
| 5 | SW | NHS-PEG | RXL-LS | X | |
| 6 | NS | NHS-POx | NU-POx | | |
| 7 | NS | NHS-POx | NU-POx | | X |
| 8 | NS | NHS-POx | NU-POx | X | X |

Patches containing granulate with P188 were fixated at 65 °C for 10 minutes to allow the P188 to melt and fixate the granulate within the carrier thereby reducing friability (i.e., loss of granulate from the carrier due to transport and/or handling). The mechanical handling characteristics were not negatively influenced by using a low temperature melting point polymeric fixative like P188.

The results obtained with these patches in the *ex-vivo* and *in-vivo* porcine bleeding models are summarised in Table 4.

**Table 4**

| **Patch** | ***Ex vivo*** | | ***In vivo*** | |
|---|---|---|---|---|
| | **Coagulation** | **Adhesion** | **Coagulation** | **Adhesion** |
| 1 | ++ | ++ | n.a. | n.a. |
| 2 | n.a. | n.a. | + | + |
| 3 | n.a. | n.a. | + | + |
| 4 | n.a. | n.a. | + | + |
| 5 | n.a. | n.a. | ++ | + |
| 6 | n.a. | n.a. | ++++ | +++ * |
| 7 | n.a. | n.a. | ++++ | ++++ ** |
| 8 | n.a. | n.a. | ++++ | ++++ ** |

| | | | | |
|---|---|---|---|---|
| *: tested on liver resections **: tested on spleen and kidney resections | | | | |

### Example 5

Haemostatic patches (SURGICEL^{®} SNoW^{™}; 5.1 x10.2 cm, average weight of 0.37 g) were impregnated with a NHS-POX/NU-POx granulate (1:1) or a powder mixture of NHS-POx powder and NU-POx powder.

The powder mixture of NHS-POx and NU-POx was prepared by mixing the two polymers as dry powders using a mortar. The molar ratio of NHS-POx to NU-POx in this powder mixture was 1:1, said molar ratio referring to the ratio of the number of NHS groups provided by NHS-POx to the number of amine groups provided by the NU-POx.

The haemostatic patches were impregnated with 1.2 grams of either the granulate or the powder mixture by shaking them in Vibratory Sieve Shaker AS 300 (Retsch) for two consecutive periods of 5 minutes each. Next, the haemostatic patches were packed in alu-alu pouches containing 1 g of silica, and vacuum sealed.

The patches were cut into 2x2 cm pieces and tested (n=3) in the ex *vivo* liver perfused model. Time to haemostasis after 1-minute pressure (TTH), haemostatic capacity as well as adhesion were determined. Results are shown in Table 5.

**Table 5**

| **NHS-POx** : **NU-POx** | ***Ex-vivo*** | | |
|---|---|---|---|
| | **TTH** | **Hemostatic capacity** | **Adhesive properties** |
| 1:1 (granulate) | 0 | +++ | ++ |
| 1:1 (dry mixing) | 0 | + | - |

## Claims

1. A biocompatible, flexible, haemostatic sheet comprising:
• a cohesive fibrous carrier structure comprising a three-dimensional interconnected interstitial space; and
• distributed within the interstitial space, a plurality of reactive polymer particles comprising (i) a water-soluble electrophilic polymer carrying at least three reactive electrophilic groups that are capable of reacting with amine groups in tissue and blood under the formation of a covalent bond and (ii) a nucleophilic cross-linking agent that contains at least two reactive nucleophilic groups that are capable of reacting with the reactive electrophilic groups of the electrophilic polymer under the formation of a covalent bond, said reactive polymer particles having a diameter in the range of 0.5-100 µm and being present in an amount of at least 3% by weight of the fibrous carrier structure.

2. Haemostatic sheet according to claim 1, wherein the fibres in the fibrous carrier structure have a mean diameter of 1-500 µm.

3. Haemostatic sheet according to claim 1 or 2, wherein the fibrous carrier structure is a felt structure, a woven structure or a knitted structure, preferably a felt structure.

4. Haemostatic sheet according to any one of the preceding claims, wherein the electrophilic polymer is selected from polyoxazolines, polyethylene glycols, polyvinylpyrrolidones, polyurethanes and combinations thereof.

5. Haemostatic sheet according to claim 4, wherein the electrophilic polymer comprising reactive electrophilic groups is a polyoxazoline.

6. Haemostatic sheet according to any one of the preceding claims, wherein the reactive electrophilic groups are selected from carboxylic acid esters, sulfonate esters, phosphonate esters, pentafluorophenyl esters, p-nitrophenyl esters, p-nitrothiophenyl esters, acid halide groups, anhydrides, ketones, aldehydes, isocyanato, thioisocyanato, isocyano, epoxides, activated hydroxyl groups, olefins, glycidyl ethers, carboxyl, succinimidyl esters, sulfo succinimidyl esters, maleimido (maleimidyl), ethenesulfonyl, imido esters, aceto acetate, halo acetal, orthopyridyl disulfide, dihydroxy-phenyl derivatives, vinyl, acrylate, acrylamide, iodoacetamide and combinations thereof.

7. Haemostatic sheet according to any one of the preceding claims, wherein the reactive polymer particles contain at least 10 wt.% of the electrophilic polymer.

8. Haemostatic sheet according to any one of the preceding claims, wherein the fibrous carrier structure comprises fibres containing a fibre polymer selected from cellulose, modified cellulose, carboxymethyldextran, poly(lactic-co-glycolic acid) (PLGA), sodium hyaluronate/carboxymethylcellulose, polyvinyl alcohol and combinations thereof.

9. Haemostatic sheet according to claim 8, wherein the fibrous carrier structure contains at least 50 wt.% of the fibre polymer.

10. Haemostatic sheet according to claim 8 or 9, wherein the fibre polymer is oxidised cellulose, preferably oxidised regenerated cellulose.

11. Haemostatic sheet according to any one of the preceding claims, wherein the nucleophilic cross-linking agent is selected from nucleophilically activated polyoxazoline; amine-functionalised polyethylene glycol; chitosan; chitosan derivatives; polyethyleneimines; polyvinylamine; polyallyl amine; amine-functionalized poly(meth)acrylates; saccharides containing amine-functional moieties; polypeptides comprising two or more amino acid residues selected from lysine, ornithine, cysteine, arginine and combinations thereof; and combinations thereof.

12. Haemostatic sheet according to claim 11, wherein the nucleophilic cross-linking agent is selected from nucleophilically activated polyoxazoline, gelatin, collagen and combinations thereof.

13. Haemostatic sheet according to any one of the preceding claims, wherein the reactive polymer particles are particle agglomerates comprising: (i) electrophilic particles containing the water-soluble electrophilic polymer; and (ii) nucleophilic particles containing the nucleophilic cross-linking agent.

14. A sealed package containing one or more haemostatic sheets according to any one of claims 1-13.

15. A method of preparing a haemostatic sheet, said method comprising:
• providing a sheet of cohesive fibrous carrier structure as defined in any one of claims 1-3;
• providing reactive polymer particles as defined in any one of claims 1, 4-7, and 11-13; and
• distributing the reactive polymer particles within the interstitial space of the fibrous carrier structure.

## Patentansprüche

1. Biokompatible, flexible, hämostatische Folie, die Folgendes umfasst:
• eine kohäsive faserige Trägerstruktur, die einen dreidimensionalen mit einander verbundenen Zwischenraum umfasst; und
• eine Vielzahl von im Zwischenraum verteilten reaktiven Polymerteilchen, die (i) ein wasserlösliches elektrophiles Polymer enthalten, das mindestens drei reaktive elektrophile Gruppen trägt, die mit Amingruppen im Gewebe und im Blut unter Bildung einer kovalenten Bindung reagieren können, und (ii) ein nukleophiles Vernetzungsmittel, das mindestens zwei reaktive nukleophile Gruppen enthält, die mit den reaktiven elektrophilen Gruppen des elektrophilen Polymers unter Bildung einer kovalenten Bindung reagieren können, wobei die besagten reaktiven Polymerteilchen einen Durchmesser im Bereich von 0,5-100 µm haben und in einer Menge von mindestens 3% berechnet nach dem Gewicht der faserigen Trägerstruktur vorhanden sind.

2. Hämostatische Folie nach Anspruch 1, wobei die Fasern in der faserigen Trägerstruktur einen mittleren Durchmesser von 1-500 µm haben.

3. Hämostatische Folie nach Anspruch 1 oder 2, wobei die faserige Trägerstruktur eine Filzstruktur, eine gewebte Struktur oder eine gestrickte Struktur ist, vorzugsweise eine Filzstruktur.

4. Hämostatische Folie nach einem der vorhergehenden Ansprüche, wobei das elektrophile Polymer aus Polyoxazolinen, Polyethylenglykolen, Polyvinylpyrrolidonen, Polyurethanen und Kombinationen davon ausgewählt ist.

5. Hämostatische Folie nach Anspruch 4, wobei das elektrophile Polymer, das reaktive elektrophile Gruppen enthält, ein Polyoxazolin ist.

6. Hämostatische Folie nach einem der vorhergehenden Ansprüche, wobei die reaktiven elektrophilen Gruppen aus Carbonsäureestern, Sulfonatestern, Phosphonatestern, Pentafluorphenylestern, p-Nitrophenylestern, p-Nitrothiophenylestern, Säurehalogenidgruppen, Anhydriden, Ketonen, Aldehyden, Isocyanato, Thioisocyanato, Isocyano, Epoxiden, aktivierten Hydroxylgruppen, Olefinen, Glycidylethern, Karboxyl, Succinimidylestern, Sulfosuccinimidylestern, Maleimido (Maleimidyl), Ethensulfonyl, Imidoestern, Acetoacetat, Halogenacetal, Orthopyridyldisulfid, Dihydroxyphenylderivaten, Vinyl, Acrylat, Acrylamid, lodacetamid und Kombinationen davon ausgewählt sind.

7. Hämostatische Folie nach einem der vorhergehenden Ansprüche, wobei die reaktiven Polymerteilchen mindestens 10 Gew.-% des elektrophilen Polymers enthalten.

8. Hämostatische Folie nach einem der vorhergehenden Ansprüche, wobei die faserige Trägerstruktur Fasern umfasst, die ein Faserpolymer enthalten, das aus Cellulose, modifizierter Cellulose, Carboxymethyldextran, Poly(milch-co-glykolsäure) (PLGA), Natriumhyaluronat/Carboxymethylcellulose, Polyvinylalkohol und Kombinationen davon ausgewählt ist.

9. Hämostatische Folie nach Anspruch 8, wobei die faserige Trägerstruktur mindestens 50 Gew.-% des Faserpolymers enthält.

10. Hämostatische Folie nach Anspruch 8 oder 9, wobei das Faserpolymer oxidierte Cellulose ist, vorzugsweise oxidierte regenerierte Cellulose.

11. Hämostatische Folie nach einem der vorhergehenden Ansprüche, wobei das nukleophile Vernetzungsmittel aus nukleophil aktiviertem Polyoxazolin; aminfunktionalisiertem Polyethylenglykol; Chitosan; Chitosanderivaten; Polyethyleniminen; Polyvinylamin; Polyallylamin; aminfunktionalisierten Poly(meth)acrylaten; Sacchariden, die aminfunktionelle Einheiten enthalten; Polypeptiden, die zwei oder mehr Aminosäurereste enthalten, die aus Lysin, Ornithin, Cystein, Arginin und Kombinationen davon ausgewählt sind; und Kombinationen davon ausgewählt ist.

12. Hämostatische Folie nach Anspruch 11, wobei das nukleophile Vernetzungsmittel aus nukleophil aktiviertem Polyoxazolin, Gelatine, Kollagen und Kombinationen davon ausgewählt ist.

13. Hämostatische Folie nach einem der vorhergehenden Ansprüche, wobei die reaktiven Polymerteilchen Teilchenagglomerate sind, die Folgendes umfassen: (i) elektrophile Teilchen, die das wasserlösliche elektrophile Polymer enthalten; und (ii) nukleophile Teilchen, die das nukleophile Vernetzungsmittel enthalten.

14. Versiegelte Verpackung, die eine oder mehrere hämostatische Folien nach einem der Ansprüche 1-13 enthält.

15. Verfahren zum Herstellen einer hämostatischen Folie, wobei das besagte Verfahren Folgendes umfasst:
• Bereitstellen einer Folie aus kohäsiver faseriger Trägerstruktur, wie in einem der Ansprüche 1-3 definiert;
• Bereitstellen reaktiver Polymerteilchen, wie in einem der Ansprüche 1, 4-7 und 11-13 definiert; und
• Verteilen der reaktiven Polymerteilchen in dem Zwischenraum der faserigen Trägerstruktur.

## Revendications

1. Feuille hémostatique biocompatible, flexible comprenant :
• une structure porteuse fibreuse cohésive comprenant un espace interstitiel tridimensionnel interconnecté ; et
• distribuées à l'intérieur de l'espace interstitiel, une pluralité de particules de polymère réactif comprenant (i) un polymère électrophile hydrosoluble portant au moins trois groupes électrophiles réactifs qui sont capables de réagir avec des groupes amine dans les tissus et le sang sous la formation d'une liaison covalente et (ii) un agent de réticulation nucléophile qui contient au moins deux groupes nucléophiles réactifs qui sont capables de réagir avec les groupes électrophiles réactifs du polymère électrophile sous la formation d'une liaison covalente, lesdites particules de polymère réactif ayant un diamètre dans la plage de 0,5-100 µm et étant présentes en une quantité d'au moins 3 % en poids de la structure de support fibreuse.

2. Feuille hémostatique selon la revendication 1, où les fibres dans la structure porteuse fibreuse ont un diamètre moyen de 1 à 500 µm.

3. Feuille hémostatique selon la revendication 1 ou 2, où la structure porteuse fibreuse est une structure en feutre, une structure tissée ou une structure tricotée, de préférence une structure en feutre.

4. Feuille hémostatique selon l'une quelconque des revendications précédentes, où le polymère électrophile est choisi parmi les polyoxazolines, les polyéthylène glycols, les polyvinylpyrrolidones, les polyuréthanes et leurs combinaisons.

5. Feuille hémostatique selon la revendication 4, où le polymère électrophile comprenant des groupes électrophiles réactifs est une polyoxazoline.

6. Feuille hémostatique selon l'une quelconque des revendications précédentes, où les groupes électrophiles réactifs sont choisis parmi esters d'acide carboxylique, esters de sulfonate, esters de phosphonate, esters de pentafluorophényle, esters de p-nitrophényle, esters de p-nitrothiophényle, groupes halogénure d'acide, anhydrides, cétones, aldéhydes, isocyanato, thioisocyanato, isocyano, époxydes, groupes hydroxyle activés, oléfines, éthers de glycidyle, carboxyles, esters de succinimidyle, esters de sulfo succinimidyle, maléimido (maléimidyle), éthènesulfonyle, imidoesters, acétoacétate, haloacétal, disulfure d'orthopyridyle, dérivés de dihydroxy-phényle, vinyle, acrylate, acrylamide, iodoacétamide et leurs combinaisons.

7. Feuille hémostatique selon l'une quelconque des revendications précédentes, où les particules de polymère réactif contiennent au moins 10 % en poids du polymère électrophile.

8. Feuille hémostatique selon l'une quelconque des revendications précédentes, où la structure porteuse fibreuse comprend des fibres contenant un polymère fibreux choisi parmi cellulose, cellulose modifiée, carboxyméthyl dextran, poly(acide lactique-co-glycolique) (PLGA), hyaluronate de sodium/carboxyméthylcellulose, alcool polyvinylique et leurs combinaisons.

9. Feuille hémostatique selon la revendication 8, où la structure porteuse fibreuse contient au moins 50 % en poids du polymère fibreux.

10. Feuille hémostatique selon la revendication 8 ou 9, où le polymère fibreux est de la cellulose oxydée, de préférence de la cellulose régénérée oxydée.

11. Feuille hémostatique selon l'une quelconque des revendications précédentes, où l'agent de réticulation nucléophile est choisi parmi polyoxazoline nucléophiliquement activée ; polyéthylène glycol fonctionnalisé par amine ; chitosane ; dérivés de chitosane ; polyéthylèneimines ; polyvinylamine; polyallylamine; poly(méth)acrylates fonctionnalisés par amine ; saccharides contenant des fractions à fonction amine ; polypeptides comprenant deux ou plusieurs résidus d'acides aminés choisis parmi lysine, ornithine, cystéine, arginine et leurs combinaisons ; et leurs combinaisons.

12. Feuille hémostatique selon la revendication 11, où l'agent de réticulation nucléophile est choisi parmi la polyoxazoline nucléophiliquement activée, la gélatine, le collagène et leurs combinaisons.

13. Feuille hémostatique selon l'une quelconque des revendications précédentes, où les particules de polymère réactif sont des agglomérats de particules comprenant : (i) des particules électrophiles contenant le polymère électrophile hydrosoluble ; et (ii) des particules nucléophiles contenant l'agent de réticulation nucléophile.

14. Emballage scellé contenant une ou plusieurs feuilles hémostatiques selon l'une quelconque des revendications 1-13.

15. Procédé de préparation d'une feuille hémostatique, ledit procédé comprenant :
• fournir une feuille de structure porteuse fibreuse cohésive telle que définie dans l'une quelconque des revendications 1 à 3 ;
• fournir des particules de polymère réactif telles que définies dans l'une quelconque des revendications 1, 4 à 7 et 11 à 13 ; et
• répartir les particules de polymère réactif au sein de l'espace interstitiel de la structure porteuse fibreuse.
